(19) Europäisches Patentamt

European Patent Office

Office européen des brevets

(11) **EP 1 262 201 B1**

(12) **EUROPEAN PATENT SPECIFICATION**

(45) Date of publication and mention
of the grant of the patent:
**08.02.2006 Bulletin 2006/06**

(51) Int Cl.:
*A61L 27/52* (2006.01)    *A61L 31/14* (2006.01)
*A61L 27/50* (2006.01)

(21) Application number: **02010715.7**

(22) Date of filing: **14.05.2002**

(54) **Variable structure element for implant devices, corresponding implant device and method of manufacturing**

Element mit variabler Struktur für implantierbare Artikel, dazugehörige implantierbare Artikel und deren Herstellung

Elément avec structure variable pour articles implantables, articles implantables y correspondant et leur procédé de préparation

(84) Designated Contracting States:
**AT BE CH CY DE DK ES FI FR GB GR IE IT LI LU MC NL PT SE TR**

(30) Priority: **18.05.2001 IT TO20010465**

(43) Date of publication of application:
**04.12.2002 Bulletin 2002/49**

(73) Proprietor: **SORIN BIOMEDICA CARDIO S.R.L.**
**13040 Saluggia (Vercelli) (IT)**

(72) Inventors:
• **Rolando, Giovanni**
**10034 Chivasso,**
**(Torino) (IT)**

• **Curcio, Maria**
**13040 Saluggia,**
**(Vercelli) (IT)**
• **Bottelli, Andrea**
**16154 Genova Sestri Ponente (IT)**

(74) Representative: **Bosotti, Luciano et al**
**c/o Buzzi, Notaro & Antonielli d'Oulx Srl,**
**Via Maria Vittoria, 18**
**10123 Torino (IT)**

(56) References cited:
**EP-A- 0 547 530        US-A- 3 991 767**
**US-A- 5 769 882**

## Description

The present invention relates in general to implantation devices and has been developed with particular attention paid to its possible application to artificial prostheses for the percutaneous treatment (low-invasive surgery) of various vascular diseases, such as peripheral vascular diseases which might arise in various anatomical sites.

Various types of vascular prostheses (grafts) are known in the art that may possibly be combined to supporting structures currently referred to as stents, such as to give rise to prostheses which are at times defined as "stent-grafts".

In actual fact, the currently used prostheses have not completely solved the problems of mechanical retainment and the consequent maintenance in situ.

There are known (see, for instance, US-A-5 489 295 and US-A-5 562 726) solutions which envisage the use, as fixing elements, of traumatic means (consisting, for example, of hooks located at the ends of the prosthesis), which are liable to damage the tissues in the area of implantation, the risk being that of causing adverse phenomena, such as, in cases of particular seriousness, internal haemorrhages that may render immediate traditional surgical treatment necessary.

A further problem is represented by the need to provide the prosthesis with a sealing system, which is localized at least at one end thereof and is likely to prevent altogether infiltration of the blood into the space between the internal wall of the vessel and the external wall of the prosthesis. This phenomenon may increase the likelihood of migration of the prosthesis and, in certain cases (for instance, in the case of prostheses used for reducing aneurysms) the aforesaid phenomenon of infiltration may lie at the root of a further, undesired expansion of the aneurysm.

In US-A-5 534 024 there is described an intraluminal prosthesis (hereinafter, the terms "intraluminal" and "vascular" will, in effect, be considered equivalent to one another, irrespective of their corresponding etymologies) which may be implanted in a blood vessel and which has a tubular structure. Specifically, it is a structure with a double wall that is able to form one or more chambers that can receive a fluid such as air in order to enable dilation and/or stiffening of the prosthesis.

Beside the intrinsic complexity of realization, the use - as the fluid for inflating or "swelling" the prosthesis - of air appears to be far from compatible with the possible use in the framework of the vascular system, in conditions where localized outflow of air, even in minimal amounts, is likely to trigger off dangerous phenomena of thrombogenesis.

Substantially similar considerations apply also to the solutions described in US-A-5 156 620, US-A-5 507 270, and US-A-5 554 180. In all cases, these are solutions based upon the formation, in the body of the prosthesis, of pockets which ought to enable expansion of the prosthesis *in situ,* by inflating or swelling it with air and/or by consolidation of the prosthesis in the position of implantation by means of injections of hardenable plastic materials.

As has already been said, the need to blow in air into a vascular site, with the possible triggering of phenomena of coagulation, renders the corresponding method of intervention somewhat critical. Considerations of an altogether similar nature also apply to the problem linked to the possible composition of the plastic materials and of the catalysts adopted for achieving hardening thereof *in situ.*

In US-A-6 117 168 there is described a multilayered tubular device, which comprises at least one first layer made of a material that is able to absorb liquid so as to increase its volume. This first layer is coupled with a layer of non-absorbent material or of a material with a lower capacity for absorption, in order to give rise to a structure that is able to expand radially as a result of the absorption of a liquid, such as a body fluid (for example, blood).

From US-A-6 159 240, there is known a device for annuloplastic surgery, which has a general ribbon-like or band-like conformation. The device is relatively rigid during manipulation and implantation, and becomes more compliant after its implantation.

Finally, in Italian patent application TO99A000218, in the name of the present applicant, there is described an intraluminal prosthesis, for instance, for the correction of aortic aneurysms, which comprises a body with a tubular wall, which can be coupled to reinforcement means for supporting the tubular wall in a splayed-out intraluminal position. The tubular wall has a structure of a fabric type for a given thickness of wall with at least one intraparietal cavity, which is delimited by the fabric-type structure and is able to receive, in a close relationship, at least one corresponding part of the reinforcement means, represented basically by one or more stent elements.

The solutions to which reference has previously been made all belong within an extremely vast line of research, as is demonstrated, not only by the documents already cited, but also, for example, by the following US patents: US-A-5 397 345, 5 104 399, 5 256 150, 5 275 622, 4 787 899, 5 507 771, 5 507 767, 5 041 126, 4 800 882, 4 580 568, 5 591 195, 5 042 707, 5 554 180, 4 655 771, 5 211 658, 4 878 906, 5 122 154, 4 562 596, 5 078 726, 4 740 207, 4 577 631, 5 456 712, 5 527 355, 5 556 414, 5 258 020, 4 512 338, 5 147 370, 5 549 635, 4 140 126, 5 123 917, 4 922 905, 5 769 887, 5 133 732, 4 886 062, 5 443 499, 5 562 725, 5 556 426, 5 522 883, 5 609 627, 4 950 227, 5 549 663, 5 639 278, 5 092 877, 5 019 090, 5 591 229, 5 571 173, 5 571 170, 5 360 443, 5 219 355, 5 464 449, 5 591 226.

For completeness of exposition, it is also advisable to mention the Italian document IT-B-1 278 360. In the latter document, there is described an intraluminal vascular prosthesis, for example for the correction of aneurysms, which comprises a tubular body provided, at least at one of its ends, with a collar part that is able to receive inside it a supporting structure, such as a stent. This solution is usually obtained by folding back on itself the body of the prosthesis at the end concerned or, else, by fixing, typically by suturing, an added, collar-type element. Further examples of these solutions are given in US-A-5 769 882, US-A-3 991 767 and EP-A-0 547 530.

The purpose of the present invention is to provide an element with a modifiable structure for implantation devices that is able to solve the problems linked to the solutions according to the prior art, described previously.

According to the present invention, the above purpose is achieved thanks to an element having the characteristics referred to specifically in the claims that follow.

The invention regards the corresponding implantation device, as well as the corresponding process of fabrication.

More specifically, the present invention relates to an element having the features set forth in the preamble of claim 1, which is known from US-A-5 769 882.

In brief, the solution according to the invention enables an artificial intraluminal prosthesis to be obtained, which is coated with biostable, biodegradable and/or bioreabsorbable synthetic polymeric material, capable of modifying its own structure by soaking, in order to guarantee an adequate mechanical tightness, a sealing action and/or a supporting action for the said prosthesis.

In particular, the solution according to the invention enables artificial intraluminal prostheses to be obtained, which have, at least in part, a porous tubular structure made of material in the form, for example, of knitted biocompatible yarn (such as, PET, PTFE, etc.), to which there is associated at least one element having a modifiable structure. The above element consists, for example, of a hydrophilic polymeric coating at the distal end and/or proximal end of the prosthesis. If this material is subjected to treatment, it gives rise to a three-dimensional lattice, and, consequently, to a structure that on the whole is an apertured one and which, in the presence of physiological liquids, is able to absorb a mass of liquid (usually water). The element in question thus presents a behaviour whereby, albeit maintaining a substantially constant volume, it assumes a good non-traumatic mechanical consistency for a desired length of time, this, in particular, according to whether it is to behave as biostable, biodegradable and/or bioreabsorbable polymer.

The aforesaid swelling (which substantially resembles the behaviour of a cavernous body, ensures a considerable mechanical tightness, at the same time guaranteeing sealing of the distal and/or proximal end of the prosthesis, thus preventing any possible infiltration of blood between the internal wall of the lumen treated and the external wall of the prosthesis.

The polymeric material used for coating the end and/or ends of the prosthesis is based on a combination of polyvinyl alcohol and a polysaccharide. The solutions which at the moment have proved most advantageous envisage the use of a polyvinyl alcohol and its mixtures with natural polymers, such as gellan and/or sodium alginate.

A polymer of demonstrated characteristics of thermosensitivity, such as to enable modification of its consistency with variation in temperature, may also be chosen.

A prosthesis according to the invention may or may not be combined with a supporting structure such as a stent. The supporting action may be increased or replaced altogether by coating the prosthesis, either partially or totally, with a polymer having the characteristics referred to previously, combined with various natural polymers and/or copolymers, e.g., hyaluronic acid, chitosan and dextran.

In the currently preferred embodiment, the solution according to the invention is based upon the physical crosslinking, consisting of repeated cycles of freezing and thawing, of the aqueous solutions of polyvinyl alcohol (PVA), which, as end result, furnish a hydrogel with high swelling capacity.

A further feature of the invention envisages the association, to the polymeric mixture, of therapeutic substances designed to prevent phenomena of thrombogenesis and/or to favour re-growth of tissue and/or the association of elasticizing substances capable of increasing the biostability of the coating described.

Even though, in the course of the ensuing description, reference will be made principally to the application to intraluminal prostheses (such as vascular prostheses), it will be appreciated that the element according to the present invention may also be used in other implantation contexts, for instance, in association with cardiac valves (with the function of prosthetic ring) and/or in association with implantation devices of the widest range of types and categories, including leads for stimulating cardiac muscle, and/or for mapping the cavities of the heart.

The present invention will now be described, purely by way of non-limiting example, with reference to the attached drawings, in which:

- Figures 1 and 2 are schematic illustrations of the modalities of formation of an element according to the present invention on an implantation device consisting of an intraluminal prosthesis;
- Figures 3 and 4 are two cross-sectional views taken along the lines III-III of Figure 1 and IV-IV of Figure 2, respectively; and
- Figures 5 to 10 illustrate, without any intention of limiting the scope, different possible variants of embodiment of a solution according to the invention.

It will be recalled that, in the most general terms, the present invention aims at providing an element having a modifiable structure which can be associated to implantation devices such as intraluminal prostheses. The modification of the structure of the element is such as to give rise to an effect of support/tightness of the implantation device as a whole, and, in particular as regards intraluminal prostheses, of the proximal and/or distal areas of the prosthesis itself.

By way of introduction to, and integration of, the ensuing detailed description, in what follows, a number of considerations are made which refer to materials of proven biocompatibility that can be used according to criteria underlying the present invention, and, in particular, for favouring anchorage of an intraluminal vascular prosthesis according to the criteria that form the basis of the invention.

Herein, materials of a synthetic and/or biological nature are considered, which are capable of forming hydrogels, or films which, in any case, are converted into hydrogels once they are hydrated. The above materials generally consist of hydrophilic-polymer molecules, which are mutually crosslinked by means of chemical bonding or other forces of cohesion.

Hydrogels

These are materials capable of withholding large quantities of water, which, however, once appropriately treated, are insoluble therein. These materials are versatile, given that they can be rendered more or less hydrophilic through the copolymerization of two or more monomers. Their capacity for absorbing water derives from the presence of hydrophilic functional groups located on the polymeric chain, such as, for example, $-OH$, $-NH_2$, $-SO_3H$, $-CONH_2$, $-COOH$. The fact that these materials are insoluble in water depends, instead, upon the presence of a three-dimensional structure due to the crosslinking between the polymer chains. They are, in fact, three-dimensional networks, in which the crosslinking points are, in general, constituted by covalent or ionic bonds.

The capacity of these materials for absorbing and withholding aqueous solutions (referred to as "swelling"), subsequently maintaining a constant volume, not only bestows on the hydrogels a marked resemblance to the highly hydrated tissues of the human body, above all from the mechanical standpoint, but also renders them permeable to small molecules such as oxygen, nutrient substances, and metabolites. The soft and rubbery consistency of the swollen hydrogels minimizes irritation of the cells and surrounding tissues due to friction, whilst the low interface tension with the aqueous solvents, due to the large quantity of liquids contained, reduces the absorption of the proteins.

PVA hydrogels

Polyvinyl alcohol (PVA), known both for its good characteristics of biocompatibility and absence of toxicity, and for its availability on the market at a low cost, is widely used for the preparation of hydrogels. Among the various techniques used for producing PVA hydrogels, one of the most interesting is the one described in the Japanese patent 57130543 in the name of Nanbu Masao, Nippon Oil Co. Ltd, "Preparation of Gel". This technique is based upon physical crosslinking, which consists in repeated cycles of freezing and thawing of the aqueous solutions of PVA. The above freezing-thawing cycles lead to formation of crystallites, which act as centres of crosslinking between the PVA chains, and, as end result, a hydrogel is obtained with high swelling capacity.

PVA-based films and natural polymers

PVA-based films and biological macromolecules of a polysaccharide nature, such as gellan and sodium alginate, can be obtained by means of the technique of evaporation of the solvent starting from aqueous solutions of the two polymers mixed in amounts such as to obtain various weight ratios.

In particular, as far as PVA-based films and gellan-based films are concerned, the characterizations on the gellan-PVA films reveal a good thermal and mechanical stability of the films obtained, which present a dense and homogeneous structure. The tests for release of PVA indicate that the treatment with glutaric aldehyde (GTA) is effective in stabilizing the material, markedly reducing the loss in water of the synthetic polymer from the various films.

Swelling tests were carried out by measuring the diameter and thickness of the films in the form of small disks both before and after the disks were dipped in water.

The above tests revealed a considerable increase in thickness of the films depending upon the composition, whilst the diameter remained practically constant for all gellan/PVA ratios higher than 20/80.

Gellan (GE) or gellan gum is the name of the extracellular polysaccharide produced by the micro-organism Sphingomonas Elodea. As it is secreted, this polysaccharide contains O-acetyl groups, which are easily removed by heat treatment with alkaline solutions.

Gellan is an anionic hetero-polysaccharide with a molecular weight of approximately $0.5 \times 10^6$ dalton. It is widely used in the food industry. In particular, in 1992 the U.S. Food and Drug Administration allowed its use as stabilizing and thickening agent, and in biotechnologies, thanks to its capacity for forming transparent gels that are acid-resistant and heat-resistant. Other applications include deodorant gels, and industrial gels and films.

The transformation of gels is due to a heat-reversible conformational transition, following upon which they pass from a state of individual disorderly macromolecules to an orderly state in which two macromolecules associate to form a double helix. One macromolecule may be involved in the formation of more than one helix. In this way, there are created areas of joining between helices with parallel alignment and consequent formation of gel. The temperature of sol-gel transition depends upon the concentration, and is around 30°C for gellan concentrations of approximately 0.5 wt%.

Gellan may be obtained, for example, from the company Sigma-Aldrich s.r.l. of Milan, under the commercial name of "Gelrite Gellan Gum". In the tests documented in what follows, the above material was developed for the preparation of the membranes and of the dippings.

Polyvinyl alcohol (PVA) is a widely available product. For instance, it can be obtained from Sigma-Aldrich s.r.1. of Milan, with a molecular weight comprised in a rather wide range, 30,000-100,000.

Experimental tests

A first set of experimental tests conducted by the applicant highlighted the fact that, in the case of the PVA- and glycerin-based system, the PVA/glycerin hydrogel with 10% PVA and glycerin 1:4 has the particularly preferential requisites for being used as coating of a prosthesis according to the invention, this both on account of its high degree of elasticity and on account of its good capacity for adhering to the surface of the prosthesis.

In the case of gellan/PVA-based mixtures, good results are obtained with the PVA/GE mixture of 70/30. In this case, however, the capacity for adhesion to the surface of the prosthesis appears to be less satisfactory.

Starting from the above results, it appears in general to be advantageous to be able to optimize the percentage of swelling (for example, in the region of 30%), thus enabling a drastic reduction in the overall dimensions of the gel prior to implantation.

As far as PVA hydrogels are concerned, it has proven useful to add gellan to the solution obtained previously, precisely in order to bestow thereon a higher percentage of swelling.

As regards PVA films, the swelling found is considerable. It is therefore important to identify a percentage ratio with the gellan that is capable of bestowing on the solution a capacity for adhesion to the surface of the prosthesis still better than the one obtained with the ratio 70/30.

In both cases, it is advantageous to have recourse to the method of application of the film or of the gel on the prosthesis through optimization of the dipping system, i.e., to identify an alternative system, or else to make a special mould.

In this connection, it should be noted that:

- the thickness of the film or swollen gel must not preferably exceed 1 mm;
- the film must preferably swell towards the outside of the implantation device;
- the element (film or gel) must not usually coat the entire graft, but only a short stretch of the end (for example, approximately 1 cm for prostheses having a diameter of 26 mm, and approximately 5 mm for prostheses having a diameter of 12 mm).

The thickness of the film or gel must be as uniform as possible along the entire circumference of the prosthesis.

PVA/gellan films of different concentrations

A solution of gellan and PVA is used in order to form a thin film capable of swelling in an aqueous solution by approximately 100-200% of its own initial thickness.

The materials used for the tests were the following:

- polyvinyl alcohol (PVA), 99 + hydrolized %, average molecular weight: 30.000-100.000;
- gellan (Gelrite Gellan Gum);
- distilled (or de-ionized) water; and
- portions of intraluminal prosthesis.

Test procedure

Two doses of one gram each of gellan and PVA are prepared.

Two 250-ml beakers are prepared with 100 ml of de-ionized water and are put on hot plates set at a temperature higher than 70°C.

The dose of PVA, together with a stirrer, is poured into one of the two beakers, with the water still cold. The solution is left to heat up by activating the stirrer in rotation up to complete dissolution of the product in the water (this occurs in the range of a couple of hours at the most).

A thermometer is set in the second beaker and, when the temperature exceeds 75°C, the dose of gellan is poured in. The time required for obtaining a good solution is in the region of three to four hours.

For each of the above solutions, samples are taken, which are poured into petri dishes for controlling dry thickness (the dishes must be set to dry under a hood without their lids on a perfectly horizontal surface).

At the same time, tubular pieces of prosthesis are prepared (polyester grafts), approximately 25 mm long, and, with the aid of a support, they are dipped, some into one solution and others into the other solution. They are then hung up to drip on the special support, after which they are put to be dried in an oven at 60°C. This operation is carried out a number of times to obtain an adequate thickness of gel on the prosthesis. The entire operation is defined as "dipping".

The samples are then subjected to analysis at the scanning electron microscope (SEM) to evaluate both their surface and their cross section.

Tests for swelling of the membranes

The samples that have been dried in the petri dishes are detached with the aid of a pair of tweezers, after which, with the aid of a mould of normalized diameter, a sample disk is prepared for each concentration. Each disk is measured to detect the effective initial thickness. Next, each disk is dipped in 10 ml of de-ionized water. The thickness is measured at different time intervals (5 min, 10 min, 20 min, 30 min, 1 h, 2h, 4 h) to assess the percentage swelling and the kinetics of the swelling.

The degree of swelling of the material in the form of the film can be expressed by the ratio between the variation in volume after the stay in water and the volume of the dry film, according to the following relation:

$$S\% = [(Vn \quad Vo)/Vo] \times 100$$

where Vo is the initial volume of the film in the dry state, Vn is the corresponding volume at the time n and S% is the percentage degree of swelling.

The measurements are made directly on the sample (each check is the average of five measurements made one after the other in slightly different positions).

The increase in the percentage of gellan with respect to PVA determines a considerable increase in the percentage of swelling of the film. In the tests conducted, percentages of swelling in the range of 100% to 400% were found. Through these results, it is thus possible to choose the most suitable solution for the particular requirements.

Vapour-phase crosslinking

In order to obtain a stable polymer, crosslinking thereof is obtained by means of a chemical treatment which envisages the use of glutaric aldehyde.

In general, the materials thus obtained are insoluble in an aqueous environment even though they conserve the capacity for absorption of considerable amounts of water.

In order to carry out the swelling tests, the coated grafts are subjected to tests altogether similar to the ones conducted for the polymer films.

Small portions of impregnated graft (squares measuring approx. 4 mm per side) were cut out, and the samples were put into petri dishes and wetted with 10 ml of de-ionized water.

At regular intervals (2.5 min, 5 min, 10 min, 30 min, 1 h, 2 h), the measurements of thickness were taken to assess the degree of swelling.

If the kinetics of variation of the thickness is carefully observed, it is noted that:

- the percentage of swelling decreases proportionally for all the different solutions tested (crosslinking does not alter the initial characteristics);
- the crosslinked solutions, after rapid growth in the first ten minutes, tend to stabilize at around half an hour, then remaining constant until the two hours have elapsed;
- the non-crosslinked solutions, instead, increase more rapidly (the peak of swelling is reached in the first ten minutes), after which the thickness tends to diminish at a constant rate.

In order to obtain a better understanding of the kinetics of the crosslinked and non-crosslinked solutions, the test was carried out on a number of graft samples coated with the same number of layers of PVA/GE 80/20. Half of the samples were crosslinked.

The swelling test followed the procedure used previously.

The crosslinked samples thus obtained were much more stable than the non-crosslinked ones, which, after the initial swelling, proved to have released substances in the 10 ml of de-ionized water in which they were dipped, which can be estimated at around 30%. In order to determine the quantity of gellan and PVA that were dissolved in water, release tests were carried out.

Test for release of PVA

The quantitative determination of the PVA was made using a method that envisages the use of iodine.

The latter, in the presence of boric acid, which acts as stabilizer, binds to the PVA, forming a coloured complex, which can be detected spectrophotometrically at 690 nm. The method has a sensitivity within a range of between 0-20 mg/l. The procedure adopted is described in what follows.

In the presence of free PVA, a greenish colouring develops immediately, which is detected at the spectrophotometer.

The solution prepared from the non-crosslinked sample underwent a colour change onto green, whilst the other solution remained of the same colour as the basic solution.

Test for release of gellan

The quantitative determination of the gellan released by the gellan/PVA film, both as such and crosslinked, was made using a spectrophotometric method known in the literature, which is based upon acidic hydrolysis of the polysaccharide.

The subsequent reaction of the monosaccharides, which is produced with cobazol, produces a purple colouring, which is detected at 530 nm.

The method has a sensitivity within a range of between 0-40 $\mu$g/ml.

The tests performed showed that the crosslinking acts on both of the elements of the solution, reducing the amount released to the limits of sensitivity of the methods of measurement.

Preparation of PVA/gellan hydrogels at different concentrations

The materials used for the preparation tests were the following:

- polyvinyl alcohol (PVA), 99 + hydrolysed %, average molecular weight: 30.000-100.000;
- gellan (Gelrite Gellan Gum);
- glycyl alcohol (glycerol or glycerin) available from Sigma Aldrich s.r.l. of Milan;
- distilled (or de-ionized) water; and
- portions of intraluminal prosthesis.

Procedure for PVA/GE 80/20 and 70/30 solutions, with glycyl alcohol in a PVA/glycerin ratio of 1:4.

For 100 ml of solution, two 5-g doses of PVA are prepared.

If a PVA/GE 80/20 solution is to be prepared, it is necessary to calculate the corresponding amount of gellan, which is obtained from the following relation:

$$5:80 = x:20$$

where x is the amount of gellan to be prepared:

$$x = (20 \times 5)/80 = 1.25 \text{ g of gellan.}$$

Consequently, 1.25 g of gellan were prepared.

In the case of the 70/30 solution, the amount of gellan to be prepared is 2.14 g. Given that the ratio between PVA and glycerin is 1:4, 20+20 g of glycerin are poured into two petri dishes.

Two 200-ml bottles with stoppers are prepared with 100 ml of de-ionized water each and are put on the hot plates set at a temperature of approximately 95°C.

As soon as the temperature of the water in the two bottles exceeds 80°C, the dose of gellan is added to each bottle.

Once the gellan has dissolved completely in the water, the PVA is added.

Once the PVA has dissolved completely in the water+gellan solution, the glycerin is added. Of these solutions, 10 ml and 5 ml are taken and put into two petri dishes for carrying out control of thickness dry.

Pieces of tubular grafts (polyester grafts), approximately 25 mm long, are prepared, and, with the aid of a support, are dipped in the respective solutions, and then hung up to drip on the special support, after which they are put to dry in an oven at 60°C. This operation is carried out a number of times to obtain an adequate thickness of gel on the prosthesis.

The hydrogels are obtained by means of the freeze-thaw method, i.e., a series of eight cycles of freezing and thawing, which bestow on the hydrogels the required characteristics of swelling.

With the exception of the first cycle, which consists in maintaining the samples in the freezer overnight at approximately 20°C (so-called "overnight cycle") and in the subsequent thawing at room temperature for approximately half an hour, the other seven cycles envisage a freezing step lasting one hour, again at -20°C, followed by a thawing step lasting 30 min at room temperature.

The hydrogels thus obtained are ready for being subjected to tests of drying-swelling in water, in order to evaluate the thickness and elasticity of the material after loss of water and subsequent rehydration.

The need to deposit a uniform layer of polymer on the surface of the prosthesis suggests the need to develop a method for application of the polymer that is alternative to mere dipping.

The method further guarantees a minimum thickness of the film on the inside of the prosthesis which will, in any case, maintain contact with the external layer of film.

The process envisages the use of a spindle, fixed to a motor, which drives it at an adjustable r.p.m., the graft being fitted onto the spindle and the two ends (approx. 5 mm per side) of the graft being left free. After the film has been spread over the outer surface of the free ends, the speed of the spindle is adjusted to the minimum r.p.m. in order to distribute the layer of film uniformly.

The layer of film deposited tends to dry in about one hour. It is thus necessary to add solution every 30 to 40 min for an adequate number of times.

Analyses carried out under a scanning electron microscope (SEM) and under an optical microscope, for example, indicate that a sample obtained after 8 depositions of polymer (at the end of which the prosthesis is put into an oven and kept at 37°C overnight to dry) reveals excellent uniformity of the polymer (approx. 10 $\mu$m) over the surface of the prosthesis.

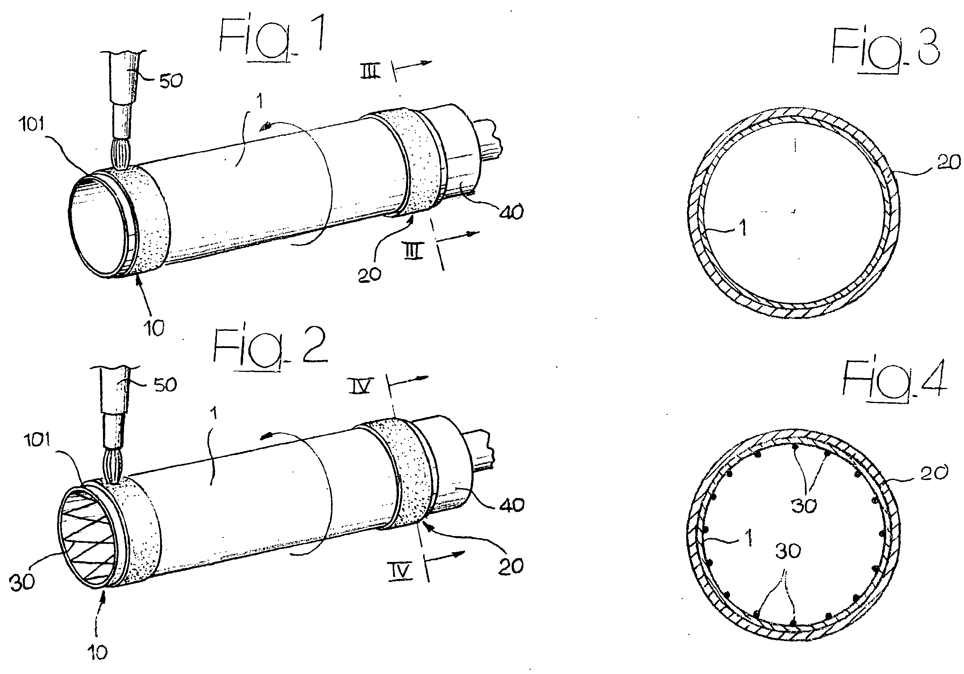

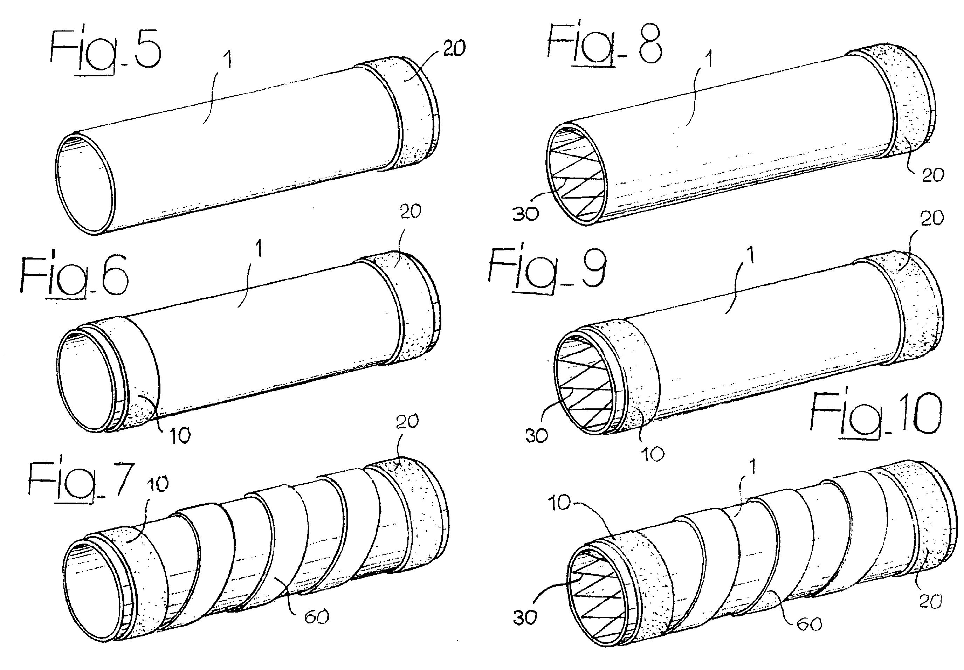

In particular, the method just described can be used to form annular supporting and/or sealing structures at one or both ends (i.e., the proximal and distal ends) of an intraluminal prosthesis, designated as a whole by 1 in Figures 1 and 2.

Precisely if account is taken of the absolute generality of the context of application of the invention, the prosthesis 1 in question is simply represented in the form of a tubular structure, hence, altogether irrespective of the specific ways in which the prosthesis may be made (which are deemed to be known to the prior art, and hence, such as not to require any detailed description herein).

In particular, the reference numbers 10 and 20 designate two band-like or ribbon-like annular formations made at the proximal end and/or the distal end of the prosthesis 1. It will be appreciated that each of the formations 10, 20 may extend exactly at the ends or else at a certain distance from the end edge or marginal edge of the corresponding end. In this connection, in both Figure 1 and Figure 2, there is noted the presence of a collar 101, which separates the annular element 10 from the homologous end of the prosthesis 1.

The reference number 40 designates the spindle referred to previously, whilst 50 designates as a whole the brush (or structurally equivalent element) used for forming the elements 10, 20.

The solution illustrated in Figures 1 and 3 is, as a whole, equivalent as regards the present invention to the one illustrated in Figures 2 and 4. The latter figures differ from Figures 1 and 3 only in that the prosthesis or implantation device 1 carries associated thereto (set inside it, in the example illustrated herein) a tubular element for dilation and support of the type currently designated as "stent". The said element is designated by 30 in the attached drawings.

Figures 5 to 10 illustrate (once again without any intention of limiting the scope of the invention) a number of possible variants of application of the solution according to the invention to intraluminal prostheses having an overall tubular structure.

In particular, Figures 5 and 6 refer to the possibility of providing a supporting/sealing element according to the invention both just at one end (see the element 20 in Figure 5) and at both ends (see the elements 10 and 20 in Figure 6) in the prosthesis 1.

Figure 7 shows that the solution according to the invention is suitable for being used not only for performing an action of support/sealing at the ends of the prosthesis 1, but also over practically the entire longitudinal development of the prosthesis 1.

In the specific case of the example illustrated in Figure 7, in addition to the end elements 10 and 20, there is envisaged the application on the prosthesis 1 of an element 60, which in itself is structurally identical to the elements 10 and/or 20 but extends throughout the entire longitudinal development of the prosthesis 1 according to a general helical path.

It is altogether evident that the development according to a helical path is here cited purely by way of example: the development could, in fact, be altogether different, comprising, for instance, an array of annular bands or ribbons basically similar to the elements 10 and 20 distributed throughout the length of the prosthesis 1, or else, a substantially continuous element which extends according to a path different from a helical path, for example, a zigzag path.

Again, the intermediate element 60 does not necessarily need to extend over the entire longitudinal development of the prosthesis 1, given that such an element may regard only one part of the overall length of the prosthesis 1. Again, in the case of a helical path (or equivalent path), the element 60 may be provided on the prosthesis 1 with a pitch different from a constant pitch (as is the case of the scheme illustrated in Figures 7 and 8), so as to present selectively variable pitches in order to render different portions of the longitudinal development of the prosthesis 1 alternatively and relatively more resistant and more compliant.

The solutions illustrated in Figures 8 to 10 are, respectively, similar to the solutions represented in Figures 5 to 7, which have just been described, with the difference represented by the presence, inside the prosthesis 1 of Figures 8 to 10, of a stent element designated as a whole by 30.

The positioning and/or spreading-out in situ of devices such as the ones illustrated in the figures of the attached drawings may be according to altogether known criteria, which consequently do not need to be recalled herein.

Of course, without prejudice to the principle of the invention, the details of construction and the embodiments may vary widely with respect to what is described and illustrated herein, without thereby departing from the scope of the present invention as defined in the ensuing claims.

## Claims

1. An element with modifiable structure for association to an implantation device (1), wherein the element is made up of a body (10, 20, 60) that is able to absorb a mass of liquid so as to assume, as a result of the absorption of said mass of liquid, a certain degree of mechanical consistency, and said body (10,20,60) is polymer-based , **characterized in that that** said body (10, 20, 60) has a polyvinyl alcohol (PVA) base and in said body (10, 20, 60), there is present in combination a polysaccharide.

2. The element according to Claim 1, **characterized in that** said body (10, 20, 60) has an apertured structure.

3. The element according to Claim 2, **characterized in that** said apertured structure is in the form of a three-dimensional lattice.

4. The element according to any one of the preceding claims, **characterized in that** said body (10, 20, 60) is configured essentially as a cavernous body.

5. The element according to any one of the preceding claims, **characterized in that**, during absorption of said mass of liquid, said body (10, 20, 60) maintains a substantially constant volume.

6. The element according to any one of the preceding claims, **characterized in that that** said body (10, 20, 60) comprises mixtures of natural polymers.

7. The element according to Claim 6, **characterized in that** said natural polymers are chosen from the group consisting of gellan, sodium alginate, hyaluronic acid, chitosan, dextran, and combinations thereof.

8. The element according to any one of the preceding claims, **characterized in that** said body (10, 20, 60) is based on a material with characteristics of thermosensitivity, so as to be able to modify its own consistency with variation of temperature.

9. The element according to Claim 1, **characterized in that** said body (10, 20, 60) is hydrogel-based.

10. The element according to Claim 9, **characterized in that** said hydrogel is obtained starting from aqueous solutions subjected to freezing and thawing.

11. The element according to any one of the preceding claims, **characterized in that** added to said body (10, 20, 60) are substances chosen from the group made up of:

   - substances able to prevent phenomena of thrombogenesis;
   - substances able to favour re-growth of tissue; and/or
   - elasticizing substances

12. The element according to Claim 1, **characterized in that** said body (10, 20, 60) is based on materials that are able to form hydrogels or films that can be transformed into hydrogels by hydration.

13. The element according to Claim 1 or to Claim 12, **characterized in that** said body (10, 20, 60) is based on hydrophilic-polymer molecules crosslinked together.

14. The element according to Claim 1 or to Claim 6, **characterized in that** said body (10, 20, 60) is constituted starting from aqueous solutions of polymers mixed in amounts such as to obtain the pre-determined weight ratios and subsequent evaporation of the solvent.

15. The element according to Claim 1, **characterized in that** said polysaccharide is chosen from among gellan, sodium alginate, and combinations thereof.

16. The element according to Claim 1, **characterized in that** said body (10, 20, 60) is based on polyvinyl alcohol and gellan.

17. The element according to any one of Claims 1, to 16, **characterized in that** in said body (10, 20, 60) there is present an addition of glycerin.

18. The element according to Claim 16, **characterized in that** the ratio between gellan and polyvinyl alcohol is between 5/95 and 20/80.

19. The element according to Claim 18, **characterized in that** said ratio is substantially 20/80.

20. The element according to Claim 1, **characterized in that** said body (10, 20, 60) is based on a polymer that has undergone crosslinking.

21. The element according to Claim 20, **characterized in that** said body (10, 20, 60) consists of a polymer that has undergone crosslinking via chemical treatment.

22. The element according to Claim 21, **characterized in that** said polymer is a polymer that has undergone crosslinking via the use of glutaric aldehyde (GTA).

23. The element according to Claim 10, **characterized in that** the preparation of said hydrogels comprises repeated cycles of freezing and thawing.

24. An implantation device, **characterized in that** it has associated thereto an element with a modifiable structure (10, 20, 60) according to any one of Claims 1 to 23.

25. The implantation device according to Claim 24, **characterized in that** said element is applied in the form of an annular structure (10, 20).

26. The implantation device according to Claim 24 or Claim 30, **characterized in that** said element with a modifiable structure is provided according to a general helical path (60).

27. The implantation device according to any one of Claims 24 to 26, **characterized in that** the device 1 comprises a prosthesis of an overall tubular shape (1) having associated thereto said element with modifiable structure (10, 20, 60) as element of consolidation and/or tightness.

28. The implantation device according to Claim 27, **characterized in that** said element with modifiable structure has an overall annular shape (10, 20) and is associated to at least one of the ends of said tubular prosthesis (1).

**29.** The implantation device according to Claim 24, **characterized in that** said element with modifiable structure (60) consists of a reinforcement which extends over the development of the body of the implantation device.

**30.** The implantation device according to any one of Claims 24 to 29, **characterized in that** said element with modifiable structure (10, 20, 60) is set, at least in part, on the outer surface of said implantation device (1).

**31.** The implantation device according to Claim 30, **characterized in that** said element with modifiable structure (10, 20) forms, on the outer surface of the implantation device (1), a sealing formation between the implantation device and the treated lumen.

**32.** The implantation device according to any one of Claims 24 to 31, **characterized in that** it further comprises a supporting structure of the stent type (30).

**33.** A process for making an implantation device according to any one of Claims 24 to 32, **characterized in that** the body (10, 20, 60) of said element with modifiable structure is applied on the implantation element by dipping.

**34.** The process for making an implantation device according to any one of Claims 24 to 32, **characterized in that** the body (10, 20, 60) of said element with modifiable structure is applied on the implantation element by spreading (50).

**35.** The process according to Claim 34, applied to an implantation device of a tubular shape, **characterized in that** it comprises the operations of:

- mounting the implantation device (1) on a rotary spindle (40);
- controlling rotation of said spindle (40) and of the implantation device (1) mounted thereon;
- providing a source (50) of supply of material which can constitute the body (10, 20, 60) of said element with modifiable structure; and
- approaching said source (50) to said implantation device (1) driven in rotation by said spindle (40), determining the application of said material on the implantation device according to a path of rotation.

**Revendications**

**1.** Elément à structure modifiable pour association avec un dispositif d'implantation (1), ledit élément étant formé d'un corps (10, 20, 60) qui est capable d'absorber une masse de liquide de manière à adopter, à la suite de l'absorption de ladite masse de liquide, un certain degré de consistance mécanique, et où ledit corps (10, 20, 60) est à base de polymère, **caractérisé en ce que** ledit corps (10, 20, 60) a une base d'alcool polyvinylique (PVA) et où, dans ledit corps (10, 20, 60), il y a, présent en combinaison un polysaccharide.

**2.** Elément selon la revendication 1, **caractérisé en ce que** ledit corps (10, 20, 60) a une structure ouverte.

**3.** Elément selon la revendication 2, **caractérisé en ce que** ladite structure ouverte se présente sous la forme d'un réseau tridimensionnel.

**4.** Elément selon l'une quelconque des revendications précédentes, **caractérisé en ce que** ledit corps (10, 20, 60) est configuré essentiellement en un corps caverneux.

**5.** Elément selon l'une quelconque des revendications précédentes, **caractérisé en ce que**, au cours de l'absorption de ladite masse de liquide, ledit corps (10, 20, 60) maintient un volume sensiblement constant.

**6.** Elément selon l'une quelconque des revendications précédentes, **caractérisé en ce que** ledit corps (10, 20, 60) comprend des mélanges de polymères naturels.

**7.** Elément selon la revendication 6, **caractérisé en ce que** lesdits polymères naturels sont choisis dans le groupe constitué de la gellane, de l'alginate de sodium, de l'acide hyaluronique, du chitosane, du dextrane et de leurs mélanges.

**8.** Elément selon l'une quelconque des revendications précédentes, **caractérisé en ce que** ledit corps (10, 20, 60) à base d'un matériau ayant des caractéristiques de thermosensibilité telles qu'elles permettent de modifier sa propre consistance lors d'une variation de température.

9. Elément selon la revendication 1, **caractérisé en ce que** ledit corps (10, 20, 60) est à base d'hydrogel.

10. Elément selon la revendication 9, **caractérisé en ce que** ledit hydrogel est obtenu à partir de solutions aqueuses soumises à une congélation et à un dégel.

11. Elément selon l'une quelconque des revendications précédentes, **caractérisé en ce que** des substances ajoutées audit corps (10, 20, 60) sont choisies dans le groupe constitué par :

    - les substances capables de prévenir les phénomènes de thrombogenèse;
    - les substances capables de favoriser la re-croissance de tissu; et/ou
    - les substances contenant de l'élasticité.

12. Elément selon la revendication 1, **caractérisé en ce que** ledit corps (10, 20, 60) est à base de matériaux qui sont capables de former des hydrogels ou des films qui peuvent être transformés en hydrogels par hydratation.

13. Elément selon la revendication 1 ou la revendication 12, **caractérisé en ce que** ledit corps (10, 20, 60) est à base de molécules de polymères hydrophiles réticulées entre elles.

14. Elément selon la revendication 1 ou la revendication 6, **caractérisé en ce que** ledit corps (10, 20, 60) est constitué à partir de solutions aqueuses de polymères mélangés en quantités telles que l'on obtienne les rapports pondéraux prédéterminés et en évaporant ensuite le solvant.

15. Elément selon la revendication 1, **caractérisé en ce que** ledit polysaccharide est choisi parmi la gellane, l'alginate de sodium et leurs mélanges.

16. Elément selon la revendication 1, **caractérisé en ce que** ledit corps (10, 20, 60) est à base d'alcool polyvinylique et de gellane.

17. Elément selon l'une quelconque des revendications 1 à 16, **caractérisé en ce qu'**il y a dans ledit corps (10, 20, 60) une addition de glycérine.

18. Elément selon la revendication 16, **caractérisé en ce que** le rapport entre la gellane et l'alcool polyvinylique est de 5/95 à 20/80.

19. Elément selon la revendication 18, **caractérisé en ce que** ledit rapport est sensiblement de 20/80.

20. Elément selon la revendication 1, **caractérisé en ce que** ledit corps (10, 20, 60) est basé sur un polymère qui a subi une réticulation.

21. Elément selon la revendication 20, **caractérisé en ce que** ledit corps (10, 20, 60) est constitué d'un polymère qui a subi une réticulation par traitement chimique.

22. Elément selon la revendication 21, **caractérisé en ce que** ledit polymère est un polymère qui a subi une réticulation par utilisation d'aldéhyde glutarique (GTA).

23. Elément selon la revendication 10, **caractérisé en ce que** la préparation desdits hydrogels comprend des cycles répétés de congélation et de dégel.

24. Dispositif d'implantation, **caractérisé en ce qu'**il lui est associé un élément à structure modifiable (10, 20, 60) selon l'une quelconque des revendications 1 à 23.

25. Dispositif d'implantation selon la revendication 24, **caractérisé en ce que** ledit élément est mis en oeuvre sous la forme d'une structure annulaire (10, 20).

26. Dispositif d'implantation selon la revendication 24 ou la revendication 30, **caractérisé en ce que** ledit élément à structure modifiable est mis en oeuvre selon un trajet hélicoïdal général (60).

**27.** Dispositif d'implantation selon l'une quelconque des revendications 24 à 26, **caractérisé en ce que** le dispositif 1 comprend une prothèse d'une forme globale tubulaire (1) à laquelle est associé ledit élément à structure modifiable (10, 20, 60) à titre d'élément de consolidation et/ou de compacité.

**28.** Dispositif d'implantation selon la revendication 27, **caractérisé en ce que** ledit élément à structure modifiable a une forme globale annulaire (10, 20) et est associé à au moins une des extrémités de ladite prothèse tubulaire (1).

**29.** Dispositif d'implantation selon la revendication 24, **caractérisé en ce que** ledit élément à structure modifiable (60) est constitué d'un renforcement qui s'étend sur l'extension du corps du dispositif d'implantation.

**30.** Dispositif d'implantation selon l'une quelconque des revendications 24 à 29, **caractérisé en ce que** ledit élément à structure modifiable (10, 20, 60) est placé, au moins en partie, sur la surface externe dudit dispositif d'implantation (1).

**31.** Dispositif d'implantation selon la revendication 30, **caractérisé en ce que** ledit élément à structure modifiable (10, 20) forme, sur la surface externe du dispositif d'implantation (1), une structure de scellage entre le dispositif d'implantation et le lumen traité.

**32.** Dispositif d'implantation selon l'une quelconque des revendications 24 à 31, **caractérisé en ce qu'**il comprend en outre une structure de support de type stent (30).

**33.** Procédé de fabrication d'un dispositif d'implantation selon l'une quelconque des revendications 24 à 32, **caractérisé en ce que** le corps (10, 20, 60) dudit élément à structure modifiable est appliqué par immersion sur l'élément d'implantation.

**34.** Procédé de fabrication d'un dispositif d'implantation selon l'une quelconque des revendications 24 à 32, **caractérisé en ce que** le corps (10, 20, 60) dudit élément à structure modifiable est appliqué par étalement sur l'élément d'implantation (50).

**35.** Procédé selon la revendication 34, appliqué à un dispositif d'implantation d'une forme tubulaire, **caractérisé en ce qu'**il comprend les opérations suivantes :

- le montage du dispositif d'implantation (1) sur une broche rotative (40);
- la commande de rotation de ladite broche (40) et du dispositif d'implantation (1) monté sur celle-ci;
- la mise en oeuvre d'une source (50) d'alimentation en matériau qui peut constituer le corps (10, 20, 60) dudit élément à structure modifiable; et
- le rapprochement de ladite source (50) dudit dispositif d'implantation (1) entraîné en rotation par ladite broche (40), en déterminant l'application dudit matériau sur ledit dispositif d'implantation selon un certain trajet de rotation.

**Patentansprüche**

**1.** Ein Element mit variabler Struktur zur Verbindung mit einem implantierbaren Artikel (1), wobei das Element aus einem Körper (1, 20, 60) besteht, der in der Lage ist, eine große Menge Flüssigkeit zu absorbieren, um als Folge der Absorption der großen Menge Flüssigkeit einen gewissen Grad an mechanischer Stabilität erhalten, und der besagte Körper (10, 20, 60) polymerbasiert ist, **dadurch gekennzeichnet, daß** der besagte Körper (10, 20, 60) eine Polyvinylalkohol (PVA)-basis hat und in dem Körper (10, 20, 60) ein Polysaccharid vorhanden ist.

**2.** Das Element nach Anspruch 1, **dadurch gekennzeichnet, daß** der Körper (10, 20, 60) eine offene Struktur aufweist.

**3.** Das Element nach Anspruch 2, **dadurch gekennzeichnet, daß** die offene Struktur die Form eines dreidimensionalen Gitters aufweist.

**4.** Das Element nach einem der vorhergehenden Ansprüche, **dadurch gekennzeichnet, daß** der Körper (10, 20, 60) im wesentlichen als Hohlkörper gestaltet ist.

**5.** Das Element nach einem der vorhergehenden Ansprüche, **dadurch gekennzeichnet, daß** während der Absorption der großen Menge Flüssigkeit der Körper (10, 20, 60) im wesentlichen ein konstantes Volumen behält.

6. Das Element nach einem der vorhergehenden Ansprüche, **dadurch gekennzeichnet, daß** der Körper (10, 20, 60) aus einer Mischung von natürlichen Polymeren besteht.

7. Das Element nach Anspruch 6, **dadurch gekennzeichnet, daß** die natürlichen Polymere aus der Gruppe bestehend aus Gellan, Natriumalginat, Hyaluronsäure, Chitosan, Dextran sowie Kombinationen von diesen gewählt sind.

8. Das Element nach einem der vorhergehenden Ansprüche, **dadurch gekennzeichnet, daß** der Körper (10, 20, 60) aus einem Material besteht, das temperaturempfindliche Eigenschaften aufweist, so daß es in der Lage ist, seine Beschaffenheit bei Variation der Temperatur zu verändern.

9. Das Element nach Anspruch 1, **dadurch gekennzeichnet, daß** der Körper hydrogelbasiert (10, 20, 60) ist.

10. Das Element nach Anspruch 9, **dadurch gekennzeichnet, daß** das Hydrogel ausgehend von wässrigen Lösungen, die einem Gefrieren und Auftauen unterworfen werden, erhalten wird.

11. Das Element nach einem der vorhergehenden Ansprüche, **dadurch gekennzeichnet, daß** dem Körper (10, 20, 60) Substanzen hinzugegeben sind, die aus folgender Gruppe ausgewählt sind:

   - Substanzen, die in der Lage sind, das Phänomen von Thrombogenesis zu verhindern;
   - Substanzen, die in der Lage sind, das Nachwachsen von Gewebe zu begünstigen; und/oder
   - verformbare Substanzen.

12. Das Element nach Anspruch 1, **dadurch gekennzeichnet, daß** der Körper (10, 20, 60) aus Materialien besteht, die in der Lage sind, Hydrogele oder Filme, die durch Hydratisierung in Hydrogele umgewandelt werden können, zu bilden.

13. Das Element nach Anspruch 1 oder 12, **dadurch gekennzeichnet, daß** der Körper (10, 20, 60) aus hydrophilen Polymermolekülen besteht, die miteinander vernetzt sind.

14. Das Element nach Anspruch 1 oder 6, **dadurch gekennzeichnet, daß** der Körper (10, 20, 60) aus wässrigen Lösungen von Polymeren, die in Mengen gemischt werden, so daß die ermittelten Gewichtsverhältnisse erhalten werden, und durch nachfolgendes Verdampfen des Lösungsmittels gebildet wird.

15. Das Element nach Anspruch 1, **dadurch gekennzeichnet, daß** das Polysaccharid aus der Gruppe Gellan, Natriumalginat und deren Kombinationen gewählt ist.

16. Das Element nach Anspruch 1, **dadurch gekennzeichnet, daß** der Körper (10, 20, 60) aus Polyvinylalkohohl und Gellan besteht.

17. Das Element nach einem der Ansprüche 1 bis 16, **dadurch gekennzeichnet, daß** der Körper (10, 20, 60) einen Zusatz Glyzerin enthält.

18. Das Element nach Anspruch 16, **dadurch gekennzeichnet, daß** das Verhältnis zwischen Gellan und Polyvinylalkohol zwischen 5/95 und 20/80 ist.

19. Das Element nach Anspruch 18, **dadurch gekennzeichnet, daß** das Verhältnis 20/80 beträgt.

20. Das Element nach Anspruch 1, **dadurch gekennzeichnet, daß** der Körper (10, 20, 60) aus einem Polymer besteht, das vernetzt worden ist.

21. Das Element nach Anspruch 20, **dadurch gekennzeichnet, daß** der Körper (10, 20, 60) aus einem Polymer besteht, das durch chemische Behandlung vernetzt worden ist.

22. Das Element nach Anspruch 21, **dadurch gekennzeichnet, daß** das besagte Polymer ein Polymer ist, das durch die Verwendung von Glutaraldehyd (GTA) vernetzt wurde.

23. Das Element nach Anspruch 10, **dadurch gekennzeichnet, daß** die Herstellung der Hydrogele wiederholte Zyklen von Gefrieren und Auftauen beinhaltet.

24. Ein implantierbarer Artikel, **dadurch gekennzeichnet, daß** mit ihm ein Element mit modifizierbarer Struktur (10, 20, 60) nach einem der Ansprüche 1 bis 23 verbunden ist.

25. Das Implantat nach Anspruch 24, **dadurch gekennzeichnet, daß** das Element in Form einer ringförmigen Struktur (10, 20) angewendet wird.

26. Das Implantat nach Anspruch 24 oder Anspruch 30, **dadurch gekennzeichnet, daß** das Element mit modifizierbarer Struktur mit einer generell helikalen Anordnung (60) versehen wird.

27. Der implantierbare Artikel nach einem der Ansprüche 24 bis 26, **dadurch gekennzeichnet, daß** die Vorrichtung (1) aus einer Prothese von insgesamt röhrenförmiger Gestalt besteht, mit der das Element mit modifizierbarer Struktur (10, 20, 60) als Element der Festigung und/oder Verstärkung verbunden ist.

28. Der implantierbare Artikel nach Anspruch 27, **dadurch gekennzeichnet, daß** das Element mit modifizierbarer Struktur eine insgesamt ringförmige Form (10, 20) besitzt und zumindest mit einem Ende der röhrenförmigen Prothese (1) verbunden ist.

29. Der implantierbare Artikel nach Anspruch 24, **dadurch gekennzeichnet, daß** das Element mit modifizierbarer Struktur (60) eine Verstärkung enthält, die sich über den Körper des implantierbaren Artikels erstreckt.

30. Der implantierbare Artikel nach einem der Ansprüche 24 bis 29, **dadurch gekennzeichnet, daß** das Element mit modifizierbarer Struktur (10, 20, 60) zumindest teilweise an der Außenfläche des implantierbaren Artikels (1) angebracht ist.

31. Der implantierbare Artikel nach Anspruch 30, **dadurch gekennzeichnet, daß** das Element mit modifizierbarer Struktur (10, 20) an der Außenfläche des implantierbaren Artikels

    (1) eine dichtende Anordnung zwischen dem implantierbaren Artikel und dem behandelnden Hohlraum bildet.

32. Der implantierbare Artikel nach einem der Ansprüche 24 bis 31, **dadurch gekennzeichnet, daß** er zusätzlich eine unterstützende Struktur von Stent-Typ (30) beinhaltet.

33. Ein Verfahren, um einen implantierbaren Artikel nach einem der Ansprüche 24 bis 32 herzustellen, **dadurch gekennzeichnet, daß** der Körper (10, 20, 60) des Elements mit modifizierbarer Struktur auf den implantierbaren Artikel durch Eintauchen aufgebracht wird.

34. Das Verfahren, um einen implantierbaren Artikel nach einem der Ansprüche 24 bis 32 herzustellen, **dadurch gekennzeichnet, daß** der Körper (10, 20, 60) des Elements mit modifizierbarer Struktur auf den implantierbaren Artikel durch Ausprägen aufgebracht wird (50).

35. Das Verfahren nach Anspruch 34, angewendet auf einen implantierbaren Artikel mit röhrenförmiger Gestalt, **dadurch gekennzeichnet, daß** es die Operationen beinhaltet:

    - Befestigung des implantierbaren Artikels (1) an einer sich drehenden Spindel (40);
    - Kontrolle der Rotation von besagter Spindel (40) und des daran befestigten implantierbaren Artikels (1);
    - Bereitstellung einer Quelle (50) zur Versorgung mit Material, das den Körper (10, 20, 60) des Elements mit modifizierbarer Struktur bilden kann; und
    - Annähern besagter Quelle (50) an den implantierbaren Artikel (1), der durch besagte Spindel (40) in Rotation versetzt wird, Festlegung der Aufbringung des besagten Materials auf den implantierbaren Artikel in Abhängigkeit von einem Rotationsweg.

Fig. 3

Fig. 4

Fig. 1

Fig. 2

Fig.-8

Fig.-9

Fig.-10

Fig.-5

Fig.-6

Fig.-7